# EUROPEAN PATENT SPECIFICATION

(11) **EP 2 397 099 B1**
(45) Date of publication and mention of the grant of the patent: **28.08.2013**
(21) Application number: 11169931.0
(22) Date of filing: 15.06.2011
(51) Int. Cl.: A61B 19/00, A61M 25/00, A61N 1/18

(54) **Optical contact sensing in medical probes**
Optischer Kontaktsensor in medizinischen Sonden
Détection de contact optique dans des sondes médicales

(30) Priority: 16.06.2010 US 816492
(43) Date of publication of application: 21.12.2011
(73) Proprietor: Biosense Webster (Israel), Ltd., Yokneam 20692 (IL)
(72) Inventor: Govari, Assaf, 34400 Haifa (IL); Altmann, Andres Claudio, 34757 Haifa (IL); Ephrath, Yaron, 35170 Karkur (IL)
(74) Representative: Small, Gary James

(56) References cited:
- EP-A2- 2 040 059
- WO-A2-2009/136311
- JP-A- 2003 164 415
- US-A1- 2009 158 852

## Description

### FIELD OF THE INVENTION

The present invention relates generally to invasive probes, and specifically to verifying contact quality between a medical probe and body tissue.

### BACKGROUND

A wide range of medical procedures involve placing objects, such as sensors, tubes, catheters, dispensing devices, and implants, within the body. Various types of sensors have been proposed for sensing the contact between a catheter and tissue in the body. Example methods and systems are described in U.S. Patent Application Publication 2007/0123750 A1.

U.S. Patent Application Publication 2009/0158852 A1 describes a contact sensing catheter system using an electromechanical contact sensor. International Patent Application Publication WO 2009/136311 A2 describes a device which uses an optical grating to determine contact pressure. European Patent Application Publication EP 2040059 A2 describes an optical tomography imaging system.

### SUMMARY OF THE INVENTION

There is provided, in accordance with an embodiment of the present invention, apparatus for contact sensing, as claimed hereinafter.

There is further provided, in accordance with an embodiment of the present invention, a computer software product as claimed hereinafter.

The present invention will be more fully understood from the following detailed description of the embodiments thereof, taken together with the drawings in which:

### BRIEF DESCRIPTION OF THE DRAWINGS

Figure 1 is a schematic, pictorial illustration of a medical system implementing optical contact sensing, in accordance with an embodiment of the present invention;
Figure 2 is a schematic, pictorial illustration showing a catheter that uses optical contact sensing, in accordance with an embodiment of the present invention;
Figure 3 is a flow diagram that schematically illustrates a method of optical contact sensing for a catheter, in accordance with an embodiment of the present invention;
Figure 4 is a circuit diagram of a multiplexing circuit, in accordance with an embodiment of the present invention; and
Figure 5 is a timing diagram illustrating control signals used in a multiplexing circuit, in accordance with an embodiment of the present invention.

### DETAILED DESCRIPTION OF EMBODIMENTS

### OVERVIEW

Various diagnostic and therapeutic procedures, such as intracardiac electrical mapping and cardiac ablation, use an invasive probe whose distal tip is fitted with at least one electrode. The electrode is typically operated when the probe is pressed against intra-body tissue. In these procedures, it is usually important to ascertain the proximity of the probe to a body cavity surface, and to determine when the distal tip of the probe is in contact with the body cavity surface

Medical probes are sometimes implemented in a loop (also referred to as "lasso") configuration, where the distal tip of the probe comprises an adjustable loop fitted with multiple electrodes. The configuration of the loop catheter enables simultaneous mapping or ablation of circumferential areas, such as a pulmonary vein. To perform the procedure effectively, however, the electrodes should be in simultaneous physical contact with the inner surface of the vein.

Embodiments of the present invention provide systems for accurate and efficient assessment of the quality of catheter-tissue contact. Assessing contact quality involves sensing actual physical contact and/or proximity between the catheter and the tissue. In some embodiments, one or more optical contact sensors are coupled to the distal tip of a catheter. Each optical sensor comprises a combination of at least one optical emitter, such as a Light Emitting Diode (LED), and at least one respective optical detector (such as a photodiode or a phototransistor) in close proximity to the emitter.

At small distances from the tissue, the optical detector senses optical radiation, which is emitted by the optical emitter and reflected from the tissue. The optical detector produces a signal that is indicative of the sensed reflection. As the optical contact sensor comes into physical contact with the tissue, the signal will increase to a maximal level. The signal produced by the optical detector is processed, so as to assess the quality of contact between the tissue and the distal end of the catheter. In an example embodiment, multiple optical contact sensors are fitted along the loop of the catheter. The signals produced by these sensors provide a high-quality assessment of the contact quality between the loop and the tissue.

The sensor configuration described herein provides a compact and efficient method to accurately and reliably assess both physical contact and proximity. Moreover, the contact quality measurements produced using these methods typically do not require calibration of individual catheters.

### SYSTEM DESCRIPTION

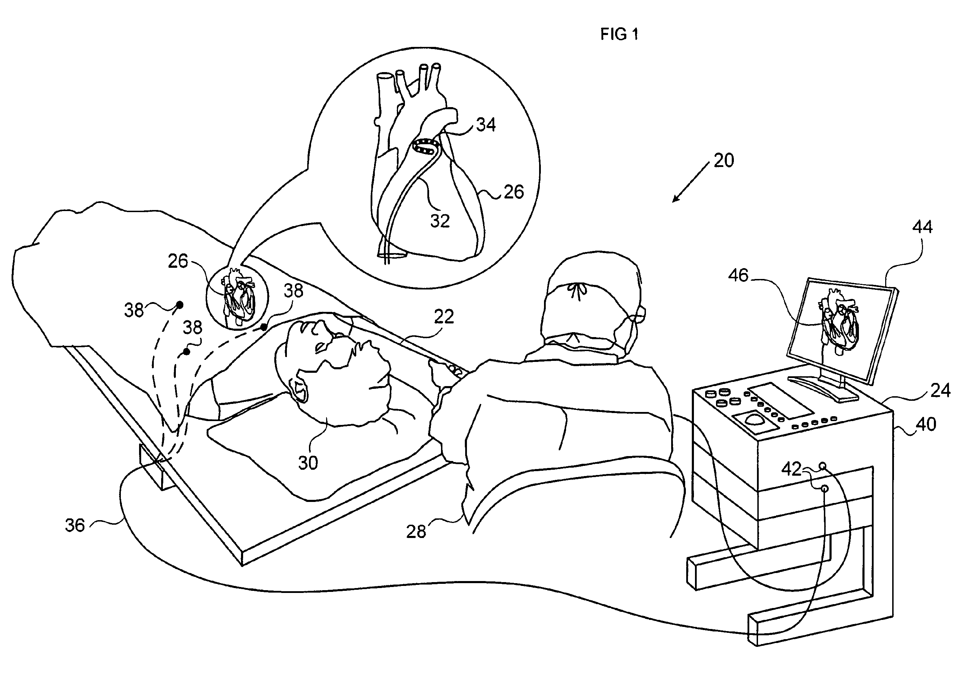

Figure 1 is a schematic, pictorial illustration of a medical system 20 that implements optical proximity sensing, in accordance with a disclosed embodiment of the present invention. System 20 comprises a probe 22, in the present example a catheter, and a control console 24. In the embodiment described hereinbelow, it is assumed that probe 22 is used for diagnostic or therapeutic treatment, such as circumferentially mapping electrical potentials in a pulmonary vein of a heart 26, or performing ablation of vein tissue. Alternatively, probe 22 may be used, *mutatis mutandis,* for other therapeutic and/or diagnostic purposes in the heart or in other body organs.

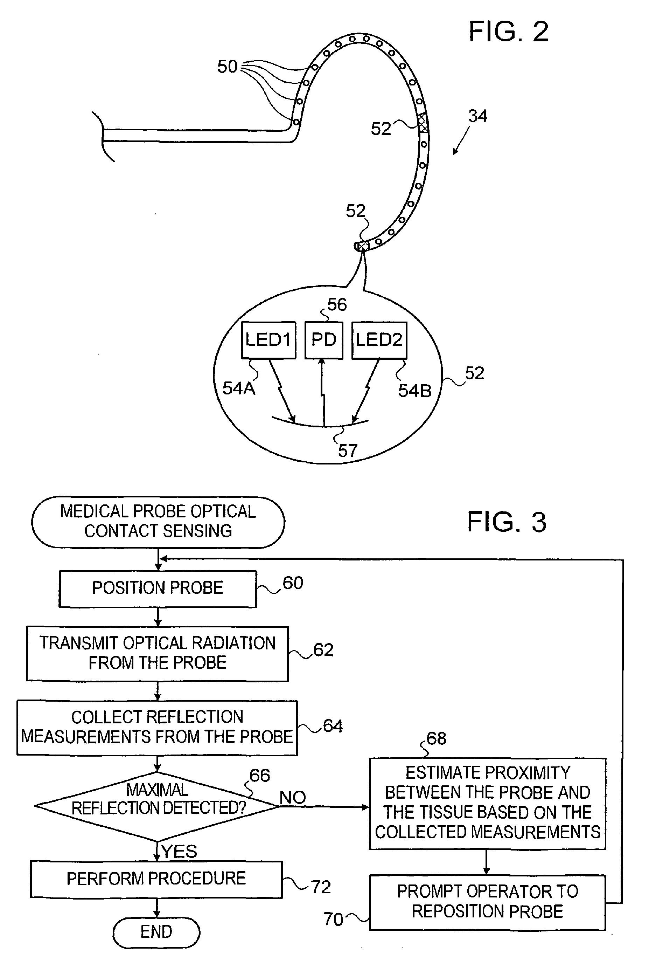

An operator 28, such as a cardiologist, inserts probe 22 through the vascular system of a patient 30 so that a distal end 32 of probe 22 enters a chamber of the patient's heart 26 (e.g., the left atrium). Operator 28 advances probe 22 so that a distal tip 34 (shown here in a "loop" configuration) engages body tissue at desired locations (e.g., vein tissue in the left superior pulmonary vein). Distal tip 34 comprises multiple electrodes and optical contact sensors. The configuration of distal tip 34 is shown in greater detail in Figure 2 below. Probe 22 is typically connected by a suitable connector at its proximal end to console 24.

Using signals from the optical contact sensors fitted in probe 22, console 24 determines the quality of contact between distal tip 34 and the vein tissue. As noted above, the term "quality of contact" refers to actual physical contact between the distal tip and the tissue, as well as proximity of the distal tip to the tissue. In the example of Fig. 1, console 24 is also connected by a cable 36 to body surface electrodes, which typically comprise adhesive skin patches 38. Console 24 determines position coordinates of probe 22 inside heart 26 based on the impedance measured between the probe and patches 38. Although system 20 measures position uses impedance-based sensors, other position tracking techniques may be used (e.g., magnetic-based sensors). Magnetic position tracking techniques are described, for example, in U.S. Patents 5,391,199, 5, 443, 489, 6, 788, 967, 6,690,963, 5,558,091, 6,172,499 6, 177, 792, Impedance-based position tracking techniques are described, for example, in U.S. Patents 5,983,126, 6,456,864 and 5,944,022,

Console 24 comprises a processor 40, which is programmed in software to carry out the functions that are described hereinbelow. Processor 40 typically comprises a general-purpose computer, with suitable front end and interface circuits for receiving signals from probe 22 and controlling the other components of console 24. The software may be downloaded to processor 40 in electronic form, over a network, for example, or it may be provided on computer-readable non-transitory tangible media, such as optical, magnetic or electronic memory media. Alternatively, some or all of the functions of processor 40 may be carried out by dedicated or programmable digital hardware components, or using a combination of hardware and software elements.

An input/output (I/O) communications interface 42 enables console 24 to interact with probe 22 and patches 38. Based on the signals received from probe 22 and from patches 38, processor 40 produces and displays a map 46 showing the position of distal tip 34 in the patient's body, the distance and/or contact indication between the loop and the body tissue, as well as status information and guidance regarding the procedure that is in progress. Map 46 is displayed to operator 28 using a display 44. The position of probe 22 may be superimposed on map 46 or on another image of heart 26.

Alternatively or additionally, system 20 may comprise an automated mechanism (not shown) for maneuvering and operating probe 22 within the body of patient 30. Such mechanisms are typically capable of controlling both the longitudinal motion (advance/retract) of probe 22 and transverse motion (deflection/steering) of distal end 32. In such embodiments, processor 40 generates a control input for controlling the motion of probe 22 based on the signals provided by the probe and the patches, as explained further hereinbelow.

Figure 2 is a schematic, pictorial illustration showing functional elements of distal tip 34 of probe 22, in accordance with an embodiment of the present invention. Distal tip 34 comprises one or more electrodes 50 and one or more optical contact sensors 52. Optical contact sensors 52 convey signals to console 24 enabling processor 40 to accurately measure both catheter-tissue contact and catheter-tissue proximity. Electrodes 50 may comprise either ablation electrodes (which perform ablation once the loop is in good contact with the tissue) or electrical mapping electrodes (which sense the electrical potential in the tissue once the loop is in good contact with the tissue). In the present example, electrodes 50 are also used for measuring the position coordinates of distal tip 34. In this embodiment, console 24 determines the position coordinates of distal tip 34 based on the measured impedance between electrodes 50 and patches 38.

Each optical contact sensor 52 comprises optical emitters 54A and 54B, such as LEDs, and an optical detector 56, such as a photodiode or a phototransistor. In the present example, each of the optical contact sensors comprises two LEDs and one photodiode. In alternative embodiments, each optical contact sensor 52 may comprise at least one optical emitter and at least one optical detector. When a given optical contact sensor 52 is at a small distance to tissue 57, detector 56 in that sensor senses reflection of the LED radiation from the tissue and outputs a signal accordingly. The signal is typically indicative of the distance between the sensor and the tissue. When the optical contact sensor comes into physical contact with the tissue, the signal from detector 56 increases to a maximal level.

LEDs 54A and 54B may emit optical radiation at different wavelengths, e.g., in the red and/or infra-red range. In the embodiment of Fig. 2, for example, LED 54A may have a certain wavelength, LED 54B may have a different wavelength, and photodiode 56 may sense reflections caused by both LEDs. By processing the reflections at the different wavelengths, processor 40 can distinguish between reflections from the vein tissue and reflections from blood cells in heart 26. As a result, the processor can assess the contact quality with the tissue with little or no distortion from blood or other reflection sources. Additionally or alternatively, console 24 may flash LEDs 54A and 54A on and off in order for processor 40 to find the exact zero level of the received signal.

Although Figure 2 shows a probe with two optical contact sensors 52 in distal tip 34, embodiments of the present invention may utilize probes with any number of optical contact sensors in the distal tip, as explained above. The number of detectors need not necessarily be equal to the number of emitters. Moreover, although Figure 2 shows a loop catheter fitted with two optical contact sensors, embodiments of the present invention may utilize any desired number of optical contact sensors fitted to a medical probe having any suitable configuration. Furthermore, the methods described hereinbelow may similarly be applied in medical procedure and measurement applications using not only loop catheters, but also catheters and probes of other types, both in the heart and in other body organs and regions.

### CONTACT QUALITY SENSING USING OPTICAL CONTACT SENSORS

As discussed supra, embodiments of the present invention provide accurate and efficient measurement of catheter-tissue physical contact, as well as catheter-tissue proximity. Based on visual feedback provided by map 46, operator 28 can then position probe 22 so that electrodes 50 are simultaneously in contact with the appropriate body surface for the medical procedure. In some embodiments, LEDs 54A and 54B emit optical radiation, and photodiode 56 conveys a signal to processor 40 indicative of optical radiation reflecting off the vein tissue. Based on the received signals, processor 40 determines the distance, or verifies contact between distal tip 34 and the tissue. In some embodiments, optical sensor 52 may be used in conjunction with another type of contact sensors (e.g., pressure/force sensors) in order to calibrate the zero level or other readings of the latter sensors.

Figure 3 is a flow diagram that schematically illustrates a method of optical contact sensing for a catheter. As operator 28 positions probe 22 in heart 26 (step 60), LEDs 54A and 54B emit optical radiation from their respective points along distal tip 34 (step 62). As discussed supra, LEDs 54A and 54B may emit optical radiation at the same or different wavelengths, and the LEDs may either be illuminated constantly or flashed on and off during use.

Photodiode 56 senses the reflected LED radiation, and produces a signal that is indicative of the intensity of the sensed reflected optical radiation. Processor 40 in console 24 accepts the signal from photodiode 56 (step 64). If photodiode 56 senses a maximal level of reflected radiation from the LEDs, then the corresponding section of distal tip 34 is most likely in direct physical contact with the vein tissue (due to the intensity of the signal). If, on the other hand, photodiode 56 senses a less than maximal level of reflected radiation from the LEDs, then the relevant section of distal tip 34 is most likely not in contact with the tissue, and the signal will have some non-zero value that is indicative of the proximity or distance between the section of the distal tip and the tissue. Due to the relationship between detected reflection and proximity, there may be a distance between photodiode 56 and the tissue where the photodiode does not detect any reflection off the tissue and generates a corresponding zero signal. The zero signal can indicate a default minimum distance, beyond which no reflection can be detected.

Processor 40 checks, based on the received signal, whether a maximal level of reflected optical radiation is detected (step 66). If a maximal reflection is not detected, processor 40 calculates the proximity of the loop catheter and the vein tissue (step 68). Since distal tip 34 will typically comprise multiple optical contact sensors 52, processor 40 will receive signals from each of these sensors, and will thus be able to determine the distance between each section of the loop catheter and the vein tissue (as well as determining which sections of the loop catheter are in good contact with the tissue). Processor 40 then updates map 46 on display 44 with the proximity information, prompts operator 28 to reposition probe 22 (step 70), and the method continues with step 60. Returning to step 66, if a maximal reflection is detected, then the loop catheter is properly positioned to perform the medical procedure (step 72).

### SIGNAL MULTIPLEXING

In some embodiments, e.g., in the above-described loop catheter configuration, the catheter distal tip is fitted with multiple optical contact sensors. Fitting the catheter with multiple sensors in addition to electrodes 50 may strain the physical dimensions of the probe because of the number of control and power supply lines passing through the catheter. In some embodiments of the present invention, the signals to and from the optical contact sensors are multiplexed onto a relatively small number of lines, thereby reducing the number of control and signal lines passing through the catheter.

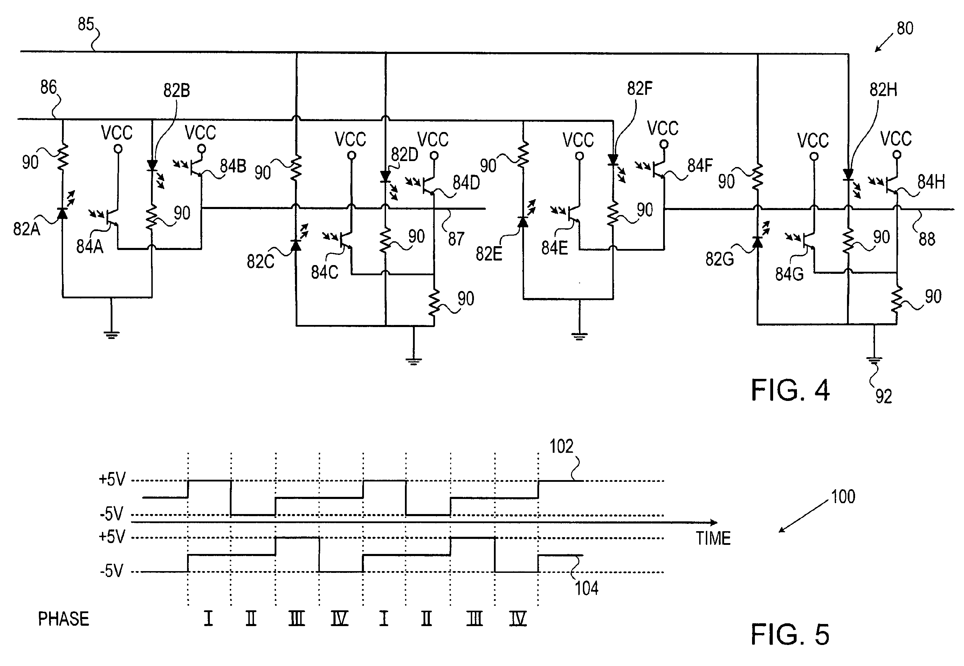

Figure 4 is a circuit diagram of a multiplexing circuit 80, in accordance with an embodiment of the present invention. Circuit 80 comprises eight LEDs 82A...82H. The optical radiation emitted by LEDs 82A...82H is sensed by phototransistors 84A...84H, respectively. Circuit 80 is controlled using a total of six lines - Two input lines, two output lines, a supply voltage line and a ground line. The eight LEDs are set alternately on and off by two input lines 85 and 86. The signals produced by the eight phototransistors are received over two output lines 87 and 88. In addition, a supply voltage (VCC) line and a ground line pass through the catheter. Circuit 80 also comprises resistors 90.

Input line 85 controls LEDs 82C, 82D, 82G and 82H. Applying a positive voltage to input line 85 activates LEDs 82D and 82H, and causes the output of phototransistors 84D and 84H to appear on output lines 87 and 88, respectively. Applying a negative voltage to input line 85 activates LEDs 82C and 82G, and causes the output of phototransistors 84C and 84G to appear on output lines 87 and 88, respectively. Applying 0V to input line 85 deactivates all four LEDs 82C, 82D, 82G and 82H.

Input line 86 controls LEDs 82A, 82B, 82E and 82F. Applying a positive voltage to input line 86 activates LEDs 82B and 82F, and causes the output of phototransistors 84B and 84F to appear on output lines 87 and 88, respectively. Applying a negative voltage to input line 86 activates LEDs 82A and 82E, and causes the output of phototransistors 84A and 84E to appear on output lines 87 and 88, respectively. Applying 0V to input line 86 deactivates all four LEDs 82A, 82B, 82E and 82F.

Figure 5 is a timing diagram 100 illustrating signal phases used to control multiplexing circuit 80, in accordance with an embodiment of the present invention. Input lines 85 and 86 are controlled using a periodic pattern having four phases. In each phase, each input line is driven with -5V, 0V or +5V. The combination of control voltages in each phase determines a pair of LEDs that will be activated during that phase (and a corresponding pair of phototransistors whose signals will be output on output lines 87 and 88).

Graphs 102 and 104 represent the voltages (+5V, 0V, -5V) applied to input lines 85 and 86, respectively. The following table shows the voltages applied to the input lines and the LEDs activated during each phase:

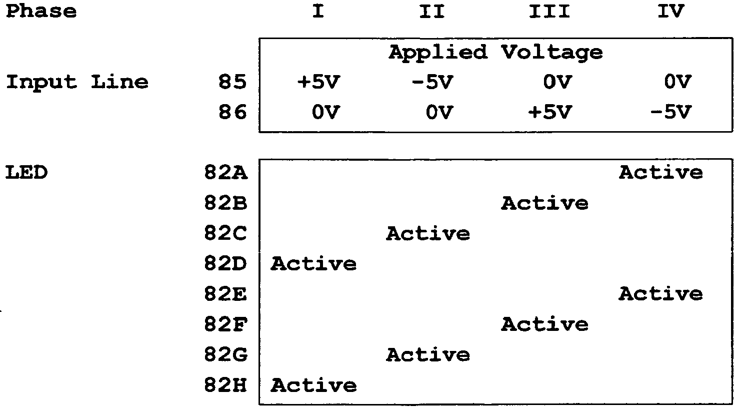

The multiplexing scheme of Figs. 4 and 5 operates eight emitter-detector pairs using only six lines. Typically, the LEDs and phototransistors are arranged so that LEDs that are active simultaneously are distant from one another. As a result, a given phototransistor is likely to sense only reflections caused by its corresponding LED. Operating two LEDs in each phase also helps to reduce DC offsets, since the current flowing in the input and output lines is substantially the same in all four phases. In alternative embodiments, any other suitable number of optical emitters and detectors can be multiplexed in any other suitable way, in order to reduce the number of lines passing through the catheter.

The corresponding structures, materials, acts, and equivalents of all means or steps plus function elements in the claims below are intended to include any structure, material, or act for performing the function in combination with other claimed elements as specifically claimed. The description of the present disclosure has been presented for purposes of illustration and description, but is not intended to be exhaustive or limiting to the disclosure in the form disclosed. Many modifications and variations will be apparent to those of ordinary skill in the art without departing from the scope of the disclosure. The embodiment was chosen and described in order to best explain the principles of the disclosure and the practical application, and to enable others of ordinary skill in the art to understand the disclosure for various embodiments with various modifications as are suited to the particular use contemplated.

It is intended that the appended claims cover all such features and advantages of the disclosure that fall within the scope of the present disclosure. As numerous modifications and changes will readily occur to those skilled in the art, it is intended that the disclosure not be limited to the limited number of embodiments described herein. Accordingly, it will be appreciated that all suitable variations, modifications and equivalents may be resorted to, falling within the scope of the present disclosure.

## Claims

1. Apparatus configured for contact sensing, comprising:
a processor (40); and
a medical probe (22) configured to be inserted into a body cavity, the probe (22) having a distal tip (34);
**characterized in that**:
the distal tip (34) of the medical probe comprises:
an optical emitter (54A), which is configured to transmit optical radiation; and
an optical detector (56), which is configured to sense a reflection of the optical radiation from tissue in the body cavity and to produce a signal indicative of the sensed reflection; and
the processor (40) is configured to receive the signal from the optical detector and to assess a quality of contact between the distal tip (34) and the tissue responsively to the signal wherein quality of contact includes physical contact between the distal tip (34) and tissue, and proximity of the distal tip (34) to tissue, and wherein the processor (40) is configured to assess the proximity by estimating a distance between the distal tip (34) and the tissue based on the received signal.

2. The apparatus according to claim 1, wherein the processor (40) is configured to assess the quality of contact by detecting a physical contact between the distal tip (34) and the tissue based on the received signal.

3. The apparatus according to claim 2, wherein the processor (40) is configured to detect the physical contact by detecting that the received signal is at a maximal level.

4. The apparatus according to claim 1, wherein the optical emitter (54A) is configured to flash the optical radiation on and off, and wherein the processor is configured to calibrate a zero level of the signal received in response to the flashed radiation.

5. The apparatus according to claim 1, and comprising an additional optical emitter (54B), wherein the optical emitter is configured to transmit a first optical radiation at a first wavelength, wherein the another optical emitter (54B) is configured to transmit a second optical radiation at a second wavelength that is different from the first wavelength, and wherein the processor (40) is configured to receive first and second signals corresponding to respective reflections of the first and second optical radiations, and distinguish between the reflection from the tissue and the reflection from blood within the cavity by processing the first and second signals.

6. The apparatus according to claim 1, and comprising a further contact sensor of a different type coupled to the distal tip (34), wherein the processor (40) is configured to calibrate the further contact sensor using the assessed quality of contact.

7. The apparatus according to claim 1, and comprising multiple optical emitters (54A; 54B) that are configured to transmit the optical radiation, and multiple optical detectors (56) that are configured to sense the reflection, wherein the processor (40) is configured to receive respective multiple signals indicative of the reflection from the multiple optical detectors, and to assess the quality of contact based on the multiple signals.

8. The apparatus according to claim 7, and comprising multiplexing circuitry, which is configured to activate at least two of the optical emitters (54A;54B) using a single input line.

9. The apparatus according to claim 7, and comprising multiplexing circuitry, which is configured to multiplex the signals from at least two of the optical emitters (54A;54B) over a single output line.

10. A computer software product, operated in conjunction with a medical probe (22) having a distal tip (34) for insertion into a body cavity, the distal tip (34) comprising an optical emitter (54A) that transmits optical radiation and an optical detector (56) that senses a reflection of the optical radiation from tissue in the body cavity and produces a signal indicative of the sensed reflection, the product comprising a non-transitory computer-readable medium, in which program instructions are stored, which instructions, when read by a computer, cause the computer to receive the signal from the optical detector (56) and to assess a quality of contact between the distal tip (34) and the tissue responsively to the signal, wherein quality of contact includes physical contact between the distal tip (34) and tissue, and proximity of the distal tip (34) to tissue, and wherein the computer is caused to assess the proximity by estimating a distance between the distal tip (34) and the tissue based on the received signal.

## Patentansprüche

1. Vorrichtung, die für eine Kontakterfassung konfiguriert ist und Folgendes umfasst:
einen Prozessor (40); und
eine medizinische Sonde (22), die konfiguriert ist, in einen Körperhohlraum eingesetzt zu werden, wobei die Sonde (22) eine distale Spitze (34) aufweist;
**dadurch gekennzeichnet, dass**:
die distale Spitze (34) der medizinischen Sonde Folgendes umfasst:
einen optischen Emitter (54A), der konfiguriert ist, optische Strahlung zu übertragen; und
einen optischen Detektor (56), der konfiguriert ist, eine Reflexion der optischen Strahlung aus dem Gewebe in dem Körperhohlraum zu erfassen und ein Signal herzustellen, das die erfasste Reflexion angibt; und
wobei der Prozessor (40) konfiguriert ist, das Signal von dem optischen Detektor zu empfangen und eine Qualität des Kontaktes zwischen der distalen Spitze (34) und dem Gewebe, das auf das Signal anspricht, zu bewerten, wobei die Qualität eines Kontakts einen physikalischen Kontakt zwischen der distalen Spitze (34) und dem Gewebe sowie die Nähe der distalen Spitze (34) zu dem Gewebe enthält, und wobei der Prozessor (40) konfiguriert ist, die Nähe zu bewerten, indem eine Entfernung zwischen der distalen Spitze (34) und dem Gewebe auf Basis des empfangenen Signals geschätzt wird.

2. Vorrichtung nach Anspruch 1, wobei der Prozessor (40) konfiguriert ist, die Qualität eines Kontakts zu bewerten, indem ein physikalischer Kontakt zwischen der distalen Spitze (34) und dem Gewebe auf Basis des empfangenen Signals detektiert wird.

3. Vorrichtung nach Anspruch 2, wobei der Prozessor (40) konfiguriert ist, den physikalischen Kontakt zu detektieren, indem detektiert wird, dass das empfangene Signal auf einer maximalen Höhe ist.

4. Vorrichtung nach Anspruch 1, wobei der optische Emitter (54A) konfiguriert ist, das Blitzlicht der optischen Strahlung an und auszuschalten, und wobei der Prozessor konfiguriert ist, einen Nullpegel des Signals, das als Reaktion auf die Blitzstrahlung empfangen worden ist, zu kalibrieren.

5. Vorrichtung nach Anspruch 1, die einen zusätzlichen optische Emitter (54B) umfasst, wobei der optische Emitter konfiguriert ist, eine erste optische Strahlung mit einer ersten Wellenlänge zu übertragen, wobei der andere optische Emitter (54B) konfiguriert ist, eine zweite optische Strahlung mit einer zweiten Wellenlänge, die verschieden von der ersten Wellenlänge ist, zu übertragen, und wobei der Prozessor (40) konfiguriert ist, ein erstes und zweites Signal entsprechend den jeweiligen Reflexionen der ersten und zweiten optischen Strahlung zu empfangen und zwischen der Reflexion aus dem Gewebe und der Reflexion aus Blut innerhalb des Hohlraums zu unterscheiden, indem das erste und zweite Signal verarbeitet werden.

6. Vorrichtung nach Anspruch 1, die einen weiteren Kontaktsensor eines anderen Typs umfasst, der an die distale Spitze (34) angekoppelt ist, wobei der Prozessor (40) konfiguriert ist, den weiteren Kontaktsensor zu kalibrieren, indem die bewertete Kontaktqualität verwendet wird.

7. Vorrichtung nach Anspruch 1, die mehrfache optische Emitter (54A; 54B), die konfiguriert sind, die optische Strahlung zu übertragen, und mehrfache optische Detektoren (56), die konfiguriert sind, die Reflexion zu erfassen, umfasst, wobei der Prozessor (40) konfiguriert ist, jeweilige mehrfache Signale zu empfangen, die die Reflexion von den mehrfachen optischen Detektoren angeben, und die Qualität eines Kontakts auf Basis der mehrfache Signale zu bewerten.

8. Vorrichtung nach Anspruch 7, die einen Multiplexschaltkreis umfasst, der konfiguriert ist, mindestens zwei der optischen Emitter (54A; 54B) unter Verwendung einer einzigen Eingangsleitung zu aktivieren.

9. Vorrichtung nach Anspruch 7, die einen Multiplexschaltkreis umfasst, der konfiguriert ist, die Signale von den mindestens zwei der optischen Emitter (54A; 54B) über eine einzige Ausgangsleitung gleichzeitig zu senden.

10. Computersoftware-Produkt, das in Verbindung mit einer medizinische Sonde (22), die eine distale Spitze (34) für eine Einsetzung in einen Körperhohlraum aufweist, eingesetzt wird, wobei die distale Spitze (34) einen optischen Emitter (54A), der optische Strahlung sendet, und einen optischen Detektor (56), der eine Reflexion der optischen Strahlung von dem Gewebe in dem Körperhohlraum erfasst und ein Signal herstellt, das die erfasste Reflexion angibt, umfasst, wobei das Produkt ein dauerhaftes computerlesbares Medium umfasst, in dem Programmanweisungen gespeichert sind, wobei die Anweisungen, wenn sie von einem Computer gelesen werden, den Computer dazu veranlassen, das Signal von dem optischen Detektor (56) zu empfangen und eine Qualität eines Kontaktes zwischen der distalen Spitze (34) und dem Gewebe, das auf das Signal anspricht, zu bewerten, wobei die Qualität eines Kontakts einen physikalischen Kontakt zwischen der distalen Spitze (34) und dem Gewebe sowie die Nähe der distalen Spitze (34) zu dem Gewebe enthält, und wobei der Computer veranlasst wird, die Nähe zu bewerten, indem eine Entfernung zwischen der distalen Spitze (34) und dem Gewebe auf Basis des empfangenen Signals geschätzt wird.

## Revendications

1. Appareil configuré pour détecter un contact, comprenant :
un processeur (40) ; et
une sonde médicale (22) configurée pour être insérée dans une cavité de corps, la sonde (22) ayant une pointe distale (34) ;
**caractérisé en ce que** :
la pointe distale (34) de la sonde médicale comprend :
un émetteur optique (54A), qui est configuré pour transmettre un rayonnement optique ; et
un détecteur optique (56), qui est configuré pour capter une réflexion du rayonnement optique provenant d'un tissu dans la cavité du corps et pour produire un signal indicatif de la réflexion captée ; et
le processeur (40) est configuré pour recevoir le signal du détecteur optique et pour évaluer une qualité de contact entre la pointe distale (34) et le tissu en réponse au signal, où une qualité de contact comprend un contact physique entre la pointe distale (34) et un tissu, et une proximité de la pointe distale (34) au tissu, et où le processeur (40) est configuré pour évaluer la proximité en estimant une distance entre la pointe distale (34) et le tissu sur la base du signal reçu.

2. Appareil selon la revendication 1, dans lequel le processeur (40) est configuré pour évaluer la qualité de contact en détectant un contact physique entre la pointe distale (34) et le tissu sur la base du signal reçu.

3. Appareil selon la revendication 2, dans lequel le processeur (40) est configuré pour détecter le contact physique en détectant que le signal reçu est à un niveau maximal.

4. Appareil selon la revendication 1, dans lequel l'émetteur optique (54A) est configuré pour émettre et éteindre le rayonnement optique, et où le processeur est configuré pour calibrer un niveau zéro du signal reçu en réponse au rayonnement émis.

5. Appareil selon la revendication 1, et comprenant un émetteur optique additionnel (54B), où l'émetteur optique est configuré pour transmettre un premier rayonnement optique à une première longueur d'onde, où l'autre émetteur optique (54B) est configuré pour transmettre un second rayonnement optique à une seconde longueur d'onde qui est différente de la première longueur d'onde, et où le processeur (40) est configuré pour recevoir des premier et second signaux correspondant aux réflexions respectives des premier et second rayonnements optiques, et faire la différence entre la réflexion provenant du tissu et la réflexion provenant du sang au sein de la cavité en traitant les premier et second signaux.

6. Appareil selon la revendication 1, et comprenant un autre capteur de contact d'un type différent couplé à la pointe distale (34), où le processeur (40) est configuré pour calibrer l'autre capteur de contact au moyen de la qualité évaluée du contact.

7. Appareil selon la revendication 1, et comprenant plusieurs émetteurs optiques (54A ; 54B) qui sont configurés pour transmettre le rayonnement optique, et plusieurs détecteurs optiques (56) qui sont configurés pour capter la réflexion, où le processeur (40) est configuré pour recevoir plusieurs signaux respectifs indicatifs de la réflexion depuis la pluralité de détecteurs optiques, et pour évaluer la qualité de contact sur la base de la pluralité de signaux.

8. Appareil selon la revendication 7, et comprenant des circuits de multiplexage, lesquels sont configurés pour activer au moins deux des émetteurs optiques (54A ; 54B) au moyen d'une ligne d'entrée unique.

9. Appareil selon la revendication 7, et comprenant des circuits de multiplexage, lesquels sont configurés pour multiplexer les signaux provenant d'au moins deux des émetteurs optiques (54A; 54B) sur une ligne de sortie unique.

10. Produit logiciel informatique, utilisé en conjonction avec une sonde médicale (22) ayant une pointe distale (34) à insérer dans une cavité de corps, la pointe distale (34) comprenant un émetteur optique (54A) qui transmet un rayonnement optique et un détecteur optique (56) qui capte une réflexion du rayonnement optique provenant du tissu dans la cavité de corps et qui produit un signal indicatif de la réflexion captée, le produit comprenant un support lisible par ordinateur non transitoire, sur lequel des instructions de programme sont stockées, lesquelles instructions, lorsqu'elles sont lues par un ordinateur entraînent que l'ordinateur reçoive le signal du détecteur optique (56) et évalue une qualité de contact entre la pointe distale (34) et le tissu en réponse au signal, où une qualité de contact comprend un contact physique entre la pointe distale (34) et un tissu, et une proximité de la pointe distale (34) au tissu, et où l'ordinateur est amené à évaluer la proximité en estimant une distance entre la pointe distale (34) et le tissu sur la base du signal reçu.
